# EUROPEAN PATENT APPLICATION

(11) **EP 2 067 768 A1**
(43) Date of publication of application: **10.06.2009**
(21) Application number: 08169835.9
(22) Date of filing: 25.11.2008
(51) Int. Cl.: C07C 227/42, C07C 229/08, C07C 243/28

(54) **A process for the preparation of (S)(+)-3-(aminomethyl)-5-methylhexanoic acid**

(30) Priority: 03.12.2007 IT MI20072262
(71) Applicant: Dipharma Francis S.r.l., 20021 Baranzate (MI) (IT)
(72) Inventor: Allegrini, Pietro, 20097 S. Donato Milanese (MI) (IT); Mantegazza, Simone, 20144 Milano (IT); Pastorello, Dario, 93012 Gela (CL) (IT); Razzetti, Gabriele, 20099 Sesto san Giovanni (MI) (IT)
(74) Representative: Minoja, Fabrizio

(57) **Abstract**

A process for the preparation of a compound of formula (I), 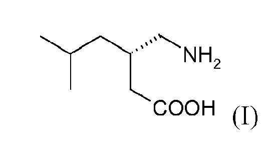 comprising:
a) the reaction of a compound of formula (II) 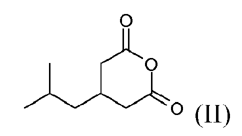 with hydrazine to obtain a compound of formula (III), 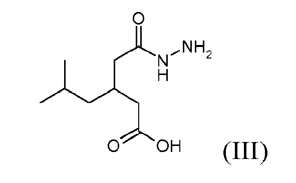 b) the conversion of a compound of formula (III) by rearrangement *via* formation of nitrene/isocyanate, in a solvent of formula R₁-OH, wherein R₁ is as herein defined, to obtain a compound of formula (IV); 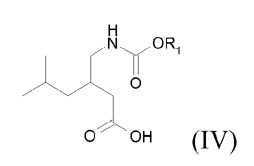 c) the enantiomeric enrichment of a compound of formula (IV) to obtain the enantiomer *(S)* of a compound of formula (V) 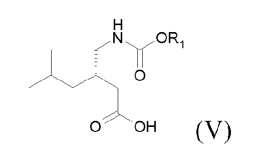 d) the hydrolysis of a compound of formula (V).

## Description

### FIELD OF THE INVENTION

The present invention relates to a novel process for the preparation of pregabalin, namely *(S)*(+)-3-(aminomethyl)-5-methylhexanoic acid, of formula (I)

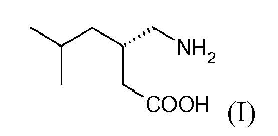

### TECHNOLOGICAL BACKGROUND

Pregabalin, disclosed in US 6,197,819, is used in the treatment of peripheral neuropathic pain, epilepsy and generalized anxiety disorder. US 5,637,767 discloses its preparation by conventional resolution of racemic 3-aminomethyl-5-methylhexanoic acid by formation of diastereomeric salts with homochiral acids or bases, separation of the diastereomeric pair by fractional crystallization or chromatography, followed by hydrolysis of the salt. Such process, however, provides pregabalin in low yields, thus affecting production times and limiting the use on an industrial scale. US 6,359,169 discloses the preparation of pregabalin through an enantioselective reaction using a chiral auxiliary, e.g. Evans oxazolidone (4*R*, 5*S*)-4-methyl-5-phenyl-2-oxazolidinone, which allows to carry out an asymmetric alkylation for introducing the desired stereocenter. Following such asymmetric alkylation, which is usually carried out at cryogenic temperatures, the comparatively expensive chiral auxiliary has to be removed, which involves higher costs and longer production times. US 2005/0283023 discloses the preparation of pregabalin by enzymatic kinetic resolution of a cyano-diester according to the following scheme:

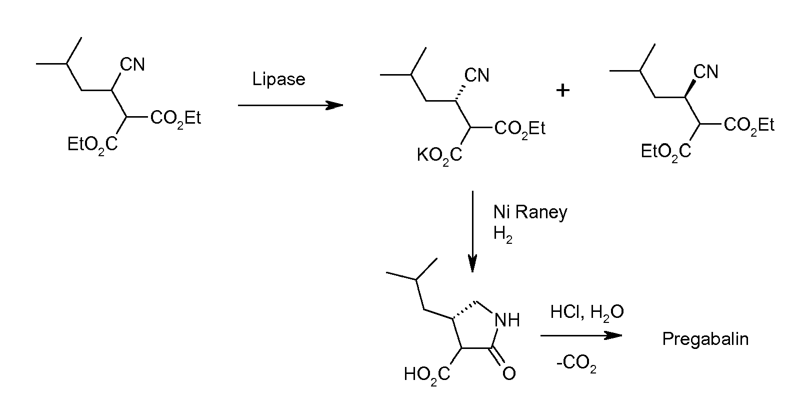

The above reported process is commercially feasible, but has some evident drawbacks, among which the use of hydrogen under pressure for the reduction of the nitrile and the use of nickel Raney, which is toxic and difficult to use.

Organic Process Research & Development 1997; 1: 26-38, reports a further synthesis of pregabalin, which however makes use of chloroform which is cancerogenic; furthermore, the last step is carried out in the presence of bromine which is toxic, corrosive, and requires dedicated apparatus and special caution on an industrial scale.

It has now been found an alternative process for the preparation of pregabalin which overcomes the drawbacks of the prior art processes. The novel process makes use of comparatively inexpensive reagents and does not require dedicated apparatus such as cryogenic reactors or high pressure hydrogenators.

### DETAILED DISCLOSURE OF THE INVENTION

An object of the invention is a process for the preparation of *(S)*(+)-3-(aminomethyl)-5-methylhexanoic acid of formula (**I**) or a salt thereof, 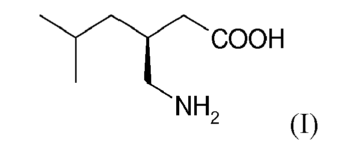 comprising:
a) the reaction of a compound of formula (II) 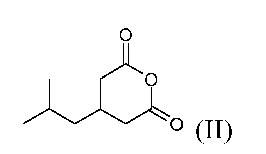 with hydrazine; if desired in the presence of a basic agent; and optionally in the presence of a solvent;
   to obtain a racemic hydrazide of formula (III), 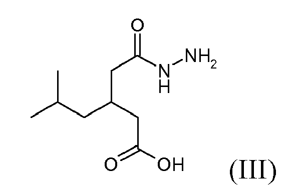
b) the conversion of a compound (III) by rearrangement *via* formation of nitrene/isocyanate in a solvent of formula R₁-OH, wherein R₁ is C₁-C₈ alkyl, aryl or aryl-C₁-C₈ alkyl, which can be optionally substituted,
   to obtain a compound of formula (IV), 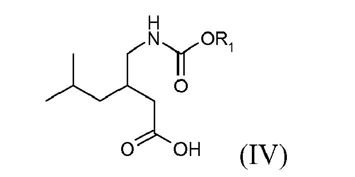 wherein R₁ is as defined above;
c) the enantiomeric enrichment of a compound of formula (IV) in the *(S)* enantiomer of formula (V); 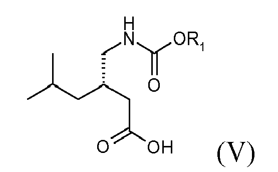 wherein R₁ is as defined above; and
d) the hydrolysis of a compound of formula (V); and, if desired, the conversion of a compound of formula (**I**) to a salt thereof, or *vice versa*.

R₁ as C₁-C₈ alkyl group is optionally substituted with 1 to 5 substituents, preferably 1 or 2, independently selected from halogen, cyano and C₁-C₆ dialkyl-amino, for example dimethyl-, diethyl-, or diisopropyl-amino. R₁ is preferably a C₁-C₄ alkyl group, more preferably methyl, ethyl, propyl, isopropyl, butyl, isobutyl or tert-butyl; in particular methyl, ethyl or i-propyl.

R₁ as aryl group is optionally substituted with 1 to 5 substituents, preferably 1, 2 or 3, independently selected from C₁-C₆ dialkyl-amino, nitro, cyano and halogen. R₁ is for example phenyl or naphthyl, in particular phenyl.

R₁ as aryl-C₁-C₈ alkyl group is optionally substituted at the aryl moiety and/or at the alkyl moiety by 1 to 5, preferably 1 or 2, substituents independently selected from halogen, nitro, cyano and C₁-C₆ dialkyl-amino, for example dimethyl-, diethyl-, or diisopropyl-amino. R₁ is, for example, phenyl-C₁-C₆ alkyl or naphthyl-C₁-C₆ alkyl, in particular phenyl-C₁-C₄ alkyl, preferably benzyl or phenylethyl.

A halogen is for example chlorine, fluorine, bromine or iodine, in particular chlorine and bromine.

An alkyl group or residue, as defined above, can be straight or branched.

In the present invention, the term "compound of formula (I), (III), (IV) or (V)" means the compound as it is or a salt thereof. Such salt is for example a pharmaceutically acceptable salt with a pharmaceutically acceptable acid or base. For example a salt with a pharmaceutically acceptable inorganic base, typically a lithium, sodium, potassium, magnesium or aluminium salt; or with an organic base, typically methylamine, triethylamine, hydrazine or phenylethylamine; or a salt with an acid selected from e.g. acetic, hydrochloric, sulfuric, methanesulfonic, propionic or camphorsulfonic acids. Said compounds can be converted to the salts thereof, or *vice versa*, according to known methods.

According to step a) of the process of the invention, a solvent can be an organic solvent selected for example from a dipolar aprotic solvent, typically dimethylformamide, dimethylacetamide, acetonitrile, dimethylsulfoxide; a ketone, typically acetone or methyl isobutyl ketone; an ether, typically tetrahydrofuran, methyl-*tert*-butyl ether or dioxane; a chlorinated solvent, typically dichloromethane; a secondary or tertiary alcohol, for example isopropanol, alcohol tert-butyl, ter-amyl alcohol; or an apolar solvent, typically toluene or hexane, or a mixture of two or more, preferably of two or three of said solvents. Alternatively the reaction can be carried out in water or mixtures of water with one or more, preferably one or two, of the solvents defined above, in a monophasic or biphasic system, typically water and isopropanol, or water and toluene. The reaction is preferably carried out in water or in a water/toluene or water/isopropanol mixture.

A basic agent can be an inorganic base, for example an alkali or alkaline-earth metal hydroxide such as sodium hydroxide, potassium hydroxide, calcium hydroxide, barium hydroxide; or an organic base, for example a tertiary amine such as triethylamine, tributylamine, diazabicycloundecene. An inorganic base is preferred, in particular sodium hydroxide.

The hydrazine can be used as free base, for example as hydrazine hydrate, or as a salt, for example the hydrochloride or sulfate, which are cleaved *in situ* in the presence of the basic agent.

The amount of hydrazine can approximately range from 0.8 to 50 mols per mole of substrate of formula (II), preferably from about 1.1 to about 20.

The reaction can be carried out at a temperature approx. ranging from -10 to 45°C, preferably from about -5 to about 10°C. The reaction times can approximately range between 20 min and 5 h.

A compound of formula (III), namely 3-hydrazinocarbonylmethyl-5-methyl-hexanoic acid, or a salt thereof, either as the individual *(R)* or *(S)* enantiomer or the mixtures thereof, is novel and is a further object of the present invention.

A compound of formula (III) can optionally be isolated as it is, or as a salt, or it can be obtained in solution and used as it is in the subsequent procedures. An aqueous or water-alcohol solution of compound of formula (III) is preferably used.

Compound of formula (II) is known.

The rearrangement of a racemic compound of formula (III), to obtain a compound of formula (IV) *via* formation of nitrene/isocyanate, can be carried out with known methods, for example following the Curtius reaction. According to the Curtius reaction, a compound of formula (III) can be reacted with a nitrosating agent, in particular an inorganic nitrite, such as sodium nitrite, or an organic nitrite such as butyl nitrite, isopropyl nitrite or isoamyl nitrite, preferably sodium nitrite, optionally in the presence of a mineral acid, in particular hydrochloric, hydrobromic or sulphuric acids, to form the corresponding acyl-azide. The acyl-azide is converted by heating to the corresponding isocyanate, which spontaneously converts to a compound of formula (IV) in the presence of a solvent of formula R₁-OH.

The formation of the acyl-azide and its conversion to the compound of formula (IV) *via* nitrene/isocyanate can be effected in separate steps. In this case, for example, the acyl-azide formation reaction can be carried out in water or in an inert solvent or mixtures thereof, at a temperature approximately ranging from -20 to 20°C, preferably from -10 to 10°C, for a time approximately ranging between 20 minutes and 40 hours, preferably from about 30 minutes to about 24 hours. The formed acyl-azide is then extracted in an inert solvent, selected from water-immiscible solvents, and contacted with a compound of formula R₁OH, wherein R₁ is as defined above, at a temperature approximately ranging from 10 to 100°C, preferably from 50 to 90°C, for a time approximately ranging from 1 to 15 hours, preferably from about 1 to about 5 hours, to give a compound of formula (IV).

An inert solvent can be for example a chlorinated solvent e.g. chloroform, dichloroethane, trichloroethane and tetrachloroethylene; an apolar solvent e.g. benzene, chlorobenzene, toluene and cyclohexane; an ester, e.g. ethyl acetate or methyl acetate; a dipolar aprotic solvent, e.g. acetonitrile, dimethylacetamide, dimethylsulfoxide, dimethyl formamide and N-methylpyrrolidone; or a ketone, e.g. acetone, ethyl ketone and methyl isobutyl ketone; an ether, e.g. dioxane, tetrahydrofuran, methyl-*tert*-butyl ether; or a mixture of two or more thereof, preferably of two or three of said solvents; preferably an inert solvent is toluene.

Alternatively, the formation of the acyl-azide and its conversion *via* nitrene/isocyanate to a compound of formula (IV) can be carried out simultaneously, for example, adding a solution of nitrite, in water or in an inert solvent as defined above or mixtures thereof, to a mixture of a compound of formula R₁OH, water or an inert solvent as defined above or mixtures thereof, hydrazide and a mineral acid or organic, for example hydrochloric, sulfuric or acetic acid. In this case, the reaction can be carried out at a temperature approximately ranging from 10 to 100°C, preferably from 50 to 90°C, for a time approximately ranging from 1 to 15 hours, preferably from about 1 to about 5 hours, to give a compound of formula (IV).

A compound of formula (IV) can be enantiomerically enriched in the *(S)* enantiomer by optical resolution through formation of a diastereoisomeric salt thereof with a resolving agent, separation of the diastereomeric couple by fractional crystallization or chromatography, followed by cleavage of the salt of the formed *(S)* enantiomer of compound of formula (V). A diastereoisomeric salt can be obtained, for example, by reaction of a compound of formula (IV) with a resolving agent, optionally in the presence of a solvent or an organic base, for example a tertiary amine, in particular triethylamine, or both. Said resolving agent can be a chiral base, typically a chiral amine, selected e.g. from those reported in *"S*.*H*. *Wilen* - *Tables of Resolving Agents and Optical Resolutions*", for example brucine, cinchonidine, cinchonine, strychnine, S-(-)-phenyl-ethyl-amine, S-(-)-naphthyl-ethyl-amine; preferably S-(-)-phenyl-ethyl-amine. A solvent can be, for example, one of the solvents cited at step a), or an ester, e.g. ethyl acetate or methyl acetate; an alcohol, e.g. methanol, ethanol or i-propanol; or a mixture of two or more, preferably of two or three of said solvents. Alternatively, the resolution can be carried out in water or mixtures of water with one or more, preferably one or two, of the solvents defined above, for example water and alcohol or water and acetone. Preferably, the resolution is carried out in water or water/alcohol mixtures or acetone or ethyl acetate.

The optical purity of a compound of formula (IV), or of the obtained diastereomeric salt, is typically equal to or higher than 98%; preferably equal to or higher than 99%.

Said purity can be optionally increased to be equal to or higher than 99.9% by means of known techniques, for example by crystallization.

Hydrolysis of a compound of formula (V) to obtain a compound of formula (I), i.e. *(S)*(+)-3-(aminomethyl)-5-methylhexanoic acid, or a salt thereof, is typically an acid hydrolysis, and can be carried out for example by treatment with a mineral acid, e.g. sulfuric acid or hydrochloric acid; in particular concentrated hydrochloric acid.

A compound of formula (I) can be converted to a salt thereof, or *vice versa*, according to known methods.

The resulting *(S)*(+)-3-(aminomethyl)-5-methylhexanoic acid has enantiomeric purity equal to or higher than the enantiomeric purity of the compound of formula (V) used as intermediate. It follows that the use of a compound of formula (V) of high enantiomeric purity, typically equal to or higher than 98%, the process of the invention provides pregabalin with an enantiomeric purity equal to or higher than 99%, which fulfils the regulatory requirements for medicaments.

The enantiomeric purity is defined as S/(S+R)x100, wherein S and R are the amount of the *(S)* and *(R)* enantiomers, respectively. According to the invention, the term *(S)* or *(R)* enantiomer means that enantiomeric purity is at least equal to approx. 96% or higher, preferably at least equal to approx. 99%.

Pregabalin obtained according to the process of the present invention has purity equal to or higher than 99.5%, preferably equal to or higher than 99.9%, which fulfils the regulatory requirements for medicaments. Pregabalin with said enantiomeric purity degree is novel and is a further object of the invention.

Pregabalin obtained according to the process of the invention has mean particle size D₅₀ ranging from 10 to 250 micrometres, which can be further reduced, for example by a fine grinding process according to known techniques, or can be increased under controlled crystallization conditions, for example by slowly cooling the solution, as it is known.

Pregabalin crystalline form obtained according to the process herein disclosed is the same as described in CN1634869A, as it can be evinced from, for example, the corresponding XRPD spectra.

A further object of the invention is a pharmaceutical composition comprising Pregabalin, or a salt thereof, with a purity equal to or higher than 99.5%, and/or enantiomeric purity equal to or higher than 98%, and a carrier and/or excipient. Said pharmaceutical composition can be prepared according to known methods in the art. Preferably in the preparation of such composition use is made of Pregabalin, or a salt thereof, having also mean particle size D₅₀ ranging from 10 to 250 micrometres.

The following examples illustrate the invention:

### Example 1 - Synthesis of 3-hydrazinocarbonylmethyl-5-methylhexanoic acid (III)

A 100 ml three-necked round-bottom flask, under nitrogen atmosphere, is added with 98% hydrazine hydrate (19.5 g, 0.382 mols), sodium hydroxide (12.4 g, 0.309 mol) in water (150 ml) and the solution is cooled to a temperature of -5°C. A solution of 3-isobutyl-glutaric anhydride (50.0 g, 0.294 mol) in toluene (200 ml) is dropped therein in about 1-2 h, keeping the temperature below 0-5°C. The mixture is reacted for about 1 h, then the phases are separated, the aqueous phase is concentrated to small volume, thereby obtaining a white solid which is taken up into isopropanol (100 ml) and filtered. The solid is dried under vacuum at a temperature of 30-35°C for 16-18 hours. 56.7 g of product are obtained, in an 86% yield.

¹H-NMR (300 MHz, D₂O, 28°C): δ 2.20-1.90 (m, 5H); 1.50 (m, 1H); 1.05 (m, 2H); 0.75 (d, 6H).

### Example 2 - Synthesis of 3-(isopropoxycarbonylamino-methyl)-5-methyl-hexanoic acid (IV; R₁= isopropyl)

A 100 ml three-necked round-bottom flask, under nitrogen atmosphere, is added with 98% hydrazine hydrate (19.5 g, 0.382 mols), sodium hydroxide (12.4 g, 0.309 mol) in water (150 ml) and the solution is cooled to a temperature of -5°C. 3-Isobutyl-glutaric anhydride (183.0 g, 1.075 mol) is dropped therein in about 1-2h, keeping the temperature below 0-5°C and the mixture is reacted for about 1h. 35-37% Hydrochloric acid (450 ml) and toluene (400 ml) are added. Keeping a temperature of -5°C, a solution of sodium nitrite (160.0 g, 2.026 mol) in water (320 ml) is added dropwise, keeping the temperature below 10-15°C. After completion of the addition, the mixture is reacted for 15-20 minutes, afterwards the phases are separated and the aqueous phase is extracted with toluene (250 ml). The cooled combined organic phases are dropped into isopropanol (800 ml) under reflux in about 1 hour. The mixture is refluxed for about 30 minutes and the solution is concentrated to small volume. The resulting oil is taken up into hexane (500 ml) and left under strong stirring for 2-3 hours, the solid is filtered and dried at 50°C for 16-18 hours. 205 g of a white solid are obtained, in a 78% yield.

¹H-NMR (300 MHz, D₂O, 28°C): δ 7.00 (broad, 1H exchange with D₂O); 4.70 (m, 1H); 3.00 (m, 1H); 2.80 (m, 1H); 2.10 (m, 2H); 1.95 (m, 1H); 1.60 (m, 1H); 1.20-1.00 (m, 8H); 0.80 (d, 6H).

### Example 3 - Synthesis of 3-(methoxycarbonyl-amino-methyl)-5-methyl-hexanoic acid (IV; R₁= methyl)

A 50 ml three-necked round-bottom flask, under nitrogen atmosphere, is added with 3-hydrazinocarbonylmethyl-5-methyl-hexanoic acid obtained according to Example 1 (3.00 g, 14.7 mmol) and 96% sulfuric acid (1.5 g, 15.4 mmol) in methanol (25 ml). The mixture is refluxed for about 5 hours, added with further 96% sulfuric acid (1.5 g, 15.4 mmol), then a solution of sodium nitrite (1.52 g, 22.0 mmol) in water (10 ml) is dropped therein at the reflux temperature, in about 30 minutes. The mixture is reacted for about 1 hour under reflux, cooled to room temperature, added with water (40 ml) and sodium hydroxide 30% sol. to pH >13. The mixture is left under stirring for about 4 hours at 40°C, then acidified to pH < 2 with 6M HCl and extracted with toluene (40 ml). The separated organic phase is concentrated to small volume under reduced pressure and the resulting oil is taken up in hexane (10 ml) and left under strong stirring for at least 3 hours. The solid is filtered and dried at room temperature under nitrogen stream: 1.5 g are obtained, in a 45% yield.
¹H-NMR (300 MHz, CDCl₃, 28°C): δ 3.6 (s, 6H); 3.2 (m, 1H); 3.0 (m, 1H); 2.3 (m, 2H); 2.1 (m, 1H); 1.6 (m, 1H); 1.2 (m, 2H); 0.9 (m, 6H).
GC-MS (M+): 231

### Example 4 - Preparation of (S)-3(isopropoxycarbonylaminomethyl)-5-methyl-hexanoic acid (V; R₁=isopropyl)

A 500 ml three-necked round-bottom flask, under nitrogen atmosphere, is added with racemic 3-(isopropoxycarbonylamino-methyl)-5-methyl-hexanoic acid (44.2 g, 0.180 mol), triethylamine (8.20 g, 0.081 mol) and *(S)*-(-)-phenyl-ethylamine (12.00 g, 0.099 mol) in a water/isopropanol 95:5 mixture (200 ml) heated at 55-60°C. The mixture is left to spontaneously cooled at room temperature, then cooled to 0-5°C for at least 1 h. The solid is filtered and washed with cold water (2 x 20 ml) then with cold toluene (4 x 20 ml), dried in a static dryer a 55-60°C for 16-18 h. 27.0 g of a white solid are obtained, in a 99:1 enantiomeric ratio.
¹H-NMR (300 MHz, CDCl₃, 28°C): δ 7.4-7.1 (m, 5H); 4.7 (m, 1H); 4.0 (q, 1H); 3.0 (dd, 1H); 2.8 (dd, 1H); 2.1 (m, 1H); 1.9 (m, 2H); 1.6 (m, 1H); 1.3 (d, 3H), 1.1 (d, 6H); 1.0 (m, 2H); 0.8 (dd, 6H).

### Example 5 - Synthesis of (S)-(+)-3-aminomethyl-5-methylhexanoic acid (I)

A 500 ml three-necked round-bottom flask, under nitrogen atmosphere, is added with *(S)*-3-(isopropoxycarbonylamino-methyl)-5-methyl-hexanoic acid *(S)*-(-)-phenyl-ethyl-amine salt (70.0 g, 0.190 mol), 35% hydrochloric acid (29.7 g, 0.285 mol), water (200 ml) and toluene (100 ml) and the mixture is vigorously stirred for 10-15 minutes. The phases are separated and the aqueous phase is extracted with toluene (2 x 100 ml). The combined organic phases are concentrated to small volume to obtain an oil which is added with 30% hydrochloric acid (57.8 g, 0.475 mol). The mixture is heated at a temperature of 90°C for 24-48 h. After completion of the reaction, 41% aqueous monomethylamine (26.7 ml) is added to pH about 6 and the mixture is left to spontaneously cool at room temperature. The mixture is cooled at 0-5°C for at least 1 h, then the solid is filtered and washed with a water/isopropanol 1:1 mixture cooled to 0-5°C (3 x 15 ml). The solid is dried in a static dryer at 50-60°C for 16-18 h. 26.6 g of a white solid are obtained, having a 99.94:0.06 enantiomeric ratio, in an 88% yield. The XRPD of the resulting product is substantially similar to that reported in CN1634869A. The product has mean particle size D₅₀ of approximately 50 micrometres.

### Example 6 - Synthesis of (S)-3(isopropoxycarbonylamino-methyl)-5-methyl-hexanoic acid (S)-(-)-phenyl-ethylamine salt

A 500 ml three-necked round-bottom flask, under nitrogen atmosphere, is added with racemic 3-(isopropoxycarbonylamino-methyl)-5-methyl-hexanoic acid (100 g, 0.205 mol), and *(S)*-(-)-phenyl-ethylamine (53.9 g, 2.18 mol) in ethyl acetate (2500 ml) and heated at 60-65°C. The mixture is cooled at room temperature at 20°C/h, and then kept at this temperature for at least 10 h. The solid is filtered and dried in a static dryer a 55-60°C for 16-18 h. The title compound (72.9 g) as a white solid is obtained (97% yield), in a 97.6 : 3.8 enantiomeric ratio. The product is recrystallized from ethyl acetate to obtain 88% yield and a 99.7 : 0.3 enantiomeric ratio.
¹H-NMR (300 MHz, CDCl₃, 28°C): δ 7.4-7.1 (m, 5H); 4.7 (m, 1H); 4.0 (q, 1H); 3.0 (dd, 1H); 2.8 (dd, 1H); 2.1 (m, 1H); 1.9 (m, 2H); 1.6 (m, 1H); 1.3 (d, 3H), 1.1 (d, 6H); 1.0 (m, 2H); 0.8 (dd, 6H).
(V; R₁= isopropyl; (S)-(-)-phenylethylamine salt)

## Claims

1. A process for the preparation of *(S)*(+)-3-(aminomethyl)-5-methylhexanoic acid, of formula (I), or a salt thereof, 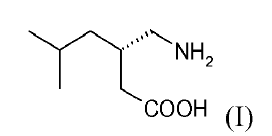 comprising:
a) the reaction of a compound of formula (II) 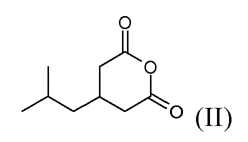 with hydrazine; if desired in the presence of a basic agent; and optionally in the presence of a solvent;
to obtain a racemic hydrazide of formula (III), 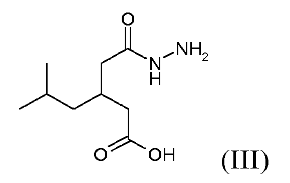
b) the conversion of a compound (III) by rearrangement *via* formation of nitrene/isocyanate in a solvent of formula R₁-OH, wherein R₁ is C₁-C₈ alkyl, aryl or aryl-C₁-C₈ alkyl, which can be optionally substituted,
to obtain a compound of formula (IV), 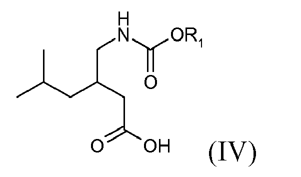 wherein R₁ is as defined above;
c) the enantiomeric enrichment of a compound of formula (IV) in the *(S)* enantiomer of formula (V); 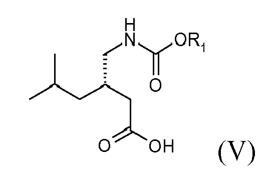 wherein R₁ is as defined above; and
d) the hydrolysis of a compound of formula (V); and, if desired, the conversion of a compound of formula (I) to a salt thereof, or *vice versa*.

2. A process as claimed in claim 1, wherein the solvent in step a) is selected from a dipolar aprotic solvent, a ketone, an ether, a chlorinated solvent, a secondary or tertiary alcohol, an apolar solvent or a mixture of two or more of said solvents; or water or a mixture of water with one or more of said solvents.

3. A process as claimed in claim 1, wherein the basic agent is selected from an alkali or alkaline-earth metal hydroxide, or a tertiary amine.

4. A process as claimed in claim 1, wherein the amount of hydrazine approximately ranges from 0.8 to 50 mols per mole of substrate of formula (II).

5. A process as claimed in claim 4, wherein the amount of hydrazine ranges from about 1.1 to about 20 mols per mole of substrate of formula (II).

6. A process as claimed in claim 1, wherein the enantiomeric enrichment is carried out by optical resolution through formation of a diastereomeric salt with a resolving agent, in the presence of a solvent and, optionally, of an organic base.

7. A process as claimed in claim 6, wherein the resolving agent is a chiral base.

8. A process as claimed in claim 6, wherein the organic base is a tertiary amine.

9. A process as claimed in claim 6, wherein the solvent is selected from a dipolar aprotic solvent, a ketone, an ether, a chlorinated solvent, a secondary or tertiary alcohol, an apolar solvent, an ester, an alcohol, or a mixture of two or more than said solvents; or water or a mixture of water with one or more of said solvents.

10. 3-Hydrazinocarbonylmethyl-5-methyl-hexanoic acid or a salt thereof.

11. 3-Hydrazinocarbonylmethyl-5-methyl-hexanoic acid, or a salt thereof, either as the individual *(R)* or *(S)* enantiomer.

12. *(S)*(+)-3-(Aminomethyl)-5-methylhexanoic acid with enantiomeric purity equal to or higher than 99%.

13. *(S)*(+)-3-(Aminomethyl)-5-acid with purity equal to or higher than 99.5%.

14. *(S)*(+)-3-(Aminomethyl)-5-methylhexanoic acid, as obtained according to the process of claim 1, having mean particle size D₅₀ ranging from 10 to 250 micrometres.
